Europäisches Patentamt

European Patent Office

Office européen des brevets

(11) Numéro de publication: **0 598 989 A1**

## (12) DEMANDE DE BREVET EUROPEEN

(21) Numéro de dépôt: **93112386.3**

(22) Date de dépôt: **03.08.93**

(51) Int. Cl.5: **A23L 1/30**, A23J 7/00, A61K 9/00, A23L 1/035

(30) Priorité: **01.09.92 CH 2735/92**

(43) Date de publication de la demande:
**01.06.94 Bulletin 94/22**

(84) Etats contractants désignés:
**AT BE CH DE DK ES FR GB GR IE IT LI LU NL PT SE**

(71) Demandeur: **SOCIETE DES PRODUITS NESTLE S.A.**
**Case postale 353**
**CH-1800 Vevey(CH)**

(72) Inventeur: **Appolonia, Corinne**
**12 Avenue Floreal**
**CH-1006 Lausanne(CH)**
Inventeur: **Masson, Gerard**
**12, Rte de Lausanne**
**CH-1096 Cully(CH)**

(74) Mandataire: **Dodin, Catherine et al**
**Avenue Nestlé 55**
**CH-1800 Vevey (CH)**

(54) **Composition nutritive et procédé de préparation.**

(57) La présente invention a trait à une composition nutritive utilisable dans l'alimentation parentérale, à laquelle on a ajouté une faible quantité d'un composé émulsifiant comprenant de la phosphatidyle choline (PC) et de la phosphatidyle ethanolanine (PE) dans un rapport PC/PE de 2 à 10.

EP 0 598 989 A1

Rank Xerox (UK) Business Services
(3.10/3.09/3.3.4)

La présente invention a trait à une composition nutritive se présentant sous forme d'une émulsion huile-dans-eau, destinée à être administrée, de préférence, par voie parentérale.

La présente invention a également trait à un procédé de préparation de cette composition nutritive.

Il est principalement connu des compositions nutritives se présentant sous forme d'émulsions huile-dans-eau et contenant 20% de lipides. Il est également connu des compositions nutritives contenant 10% de lipides. Ces compositions nutritives, destinées à l'alimentation parentérale, doivent répondre à des critères stricts, en particulier doivent être parfaitement stériles.

Or, il est connu que la stérilisation a notamment pour effet de déstabiliser les émulsions huile-dans-eau, qui vont alors se séparer en deux, voire plusieurs, phases.

Pour remédier à ce problème, on ajoute généralement un émulsifiant à l'émulsion lors de sa préparation.

On ajoute, en particulier, de la lécithine, à raison de 1,2% à 2% en poids, ce qui permet de stériliser la composition nutritive sans entraîner de déstabilisation.

Ceci est, par exemple, illustré dans l'article de Masson et al. publié dans la Revue Française des Corps Gras (oct.1984) 10, 391-394.

Or, dans le cas des compositions nutritives contenant peu de lipides, la présence d'une quantité importante de phospholipides libres, composants majoritaires de la lécithine, présente des inconvénients. Ceci est plus particulièrement remarquable dans le cas des émulsions à 10%, dans lesquelles le rapport phospholipides/lipides peut être de l'ordre de 10 à 15 pour 100.

Il a été constaté que, lorsqu'il y a dans une émulsion trop de phospholipides libres, ceux-ci ont tendance à former des micelles qui ont pour effet de déstabiliser encore plus l'émulsion, notamment lors de la stérilisation.

D'autre part, et ce problème est abordé dans l'article de Haumont et al., publié dans "The Journal of Pediatrics" (1989) 115 787-793, il a été constaté que, lorsque l'on fait ingérer à des nourrissons une émulsion à 10% contenant 1,2% de lécithine, la concentration en triglycérides du plasma sanguin augmente considérablement en comparaison avec l'augmentation obtenue si l'on utilise une émulsion à 20%. On constate également une accumulation de cholestérol et de lipoprotéines à faible densité dans le sang.

La présente invention a pour but de pallier ces inconvénients et de proposer une composition nutritive, stable même après stérilisation, qui contient une quantité très faible de composé émulsifiant.

La présente invention a donc pour objet une composition se présentant sous forme d'une émulsion huile-dans eau, comprenant, en poids, au moins 10% de lipides et au moins 0,4% d'un composé émulsifiant comprenant de la phosphatidyle choline (PC) et de la phosphatidyle éthanolamine (PE), en un rapport PC/PE compris entre 2 et 10.

Un autre objet de l'invention est un procédé de préparation de ladite composition, dans lequel on prépare une phase aqueuse et une phase lipidique, on ajoute à l'une et/ou l'autre des phases au moins 0,4% d'un composé émulsifiant comprenant de la phosphatidyle choline (PC) et de la phosphatidyle éthanolamine (PE), en un rapport PC/PE compris entre 2 et 10, on mélange les deux phases résultantes de manière à obtenir une émulsion comprenant au moins 10% en poids de lipides, et l'on stérilise l'émulsion obtenue.

On a en effet constaté que l'utilisation d'un composé émulsifiant comprenant de la phosphatidyle choline et de la phosphatidyle éthanolamine, en un rapport PC/PE compris entre 2 et 10, permet de diminuer de façon remarquable la quantité d'émulsifiant à ajouter, en particulier dans des compositions contenant peu de lipides, telles que les émulsions à 10%.

Cette utilisation peut également permettre l'addition à la-dite composition d'acides aminés, sans entraîner de déstabilisation lors de la stérilisation.

Encore un autre avantage est de permettre la préparation de compositions nutritives sans odeur et/ou couleur indésirables, ceci étant dû à la faible quantité de composé émulsifiant ajouté.

Par stabilité, on entend la propriété qu'à une émulsion de ne présenter aucune tendance à la séparation des phases ou de ses composants.

Les caractéristiques et avantages de la présente invention ressortiront de la description qui suit, dans laquelle les parties et les pourcentages sont donnés en poids, et les abréviations suivantes sont utllisées :

. PC : phosphatidyle choline

. PE : phosphatidyle éthanolamine

Pour mettre en oeuvre le procédé selon l'invention, on prépare une émulsion huile-dans-eau, constituée par un mélange d'une phase aqueuse et d'une phase lipidique.

La phase lipidique peut être constituée d'huiles d'origine végétale telles que les huiles de palme, de maïs, de canola, de soja, d'olive et de tournesol, ou d'origine animale telles que l'huile de beurre ou de poisson, ou encore de triglycérides à chaîne moyenne. On peut également utiliser un mélange de ces différentes huiles.

La phase aqueuse est principalement constituée d'eau, de préférence distillée ou déminéralisée, à laquelle on peut ajouter des glucides.

On peut choisir les glucides dans un groupe constitué par les mono ou polysaccharides tels que le maltose, le fructose ou le glucose, les maltodextrines dont l'équivalent dextrose (DE) est compris entre 10 et 50, de préférence proche de 45, et les polyalcools tels que le glycérol, le sorbitol ou le xylitol.

La présente émulsion est particulièrement remarquable par le fait qu'elle comprend au moins 10% de lipides et au moins 0,4% d'un composé émulsifiant comprenant de la phosphatidyle choline et de la phosphatidyle éthanolamine, en un rapport PC/PE compris entre 2 et 10.

Ce composé émulsifiant peut être ajouté, en fonction de sa solubilité, à la phase aqueuse ou à la phase lipidique, avant leur mélange.

Afin de préparer l'émulsion huile-dans-eau, on peut chauffer, de manière séparée, les phases aqueuse et lipidique jusqu'à une température de 40-60°C, tout en maintenant une agitation constante, le chauffage étant de préférence effectué sous gaz inerte, par exemple sous azote, de manière à éviter les contaminations éventuelles ou l'oxydation des matières grasses.

On peut alors mélanger les deux phases, par exemple par addition de 10 à 40 parties de phase lipidique à 100 parties de phase aqueuse, tout en maintenant l'agitation constante et la température à une valeur de 40-60°C.

Afin d'améliorer la stabilité de l'émulsion obtenue, on peut réduire la taille moyenne des gouttelettes de la phase lipidique, par exemple par homogénéisation. Ceci permet également d'assurer une distribution des tailles de particules la plus étroite possible.

Pour ce faire, on peut pré-homogénéiser l'émulsion sous une pression de 15-25 bar, afin d'obtenir une taille moyenne des gouttes de l'ordre de 1,5-2,5 $\mu$m, puis homogénéiser l'émulsion, par exemple dans un homogénéisateur à haute pression de l'ordre de 200-1200 bar.

Cette dernière étape peut être répétée plusieurs fois, afin d'obtenir une émulsion dont la taille moyenne des gouttelettes de la phase lipidique est de l'ordre de 0,15-0,35 $\mu$m.

De préférence, l'homogénéisation est effectuée à 40-60°C. L'émulsion homogénéisée est ensuite maintenue sous agitation constante et sous gaz inerte tel que l'azote.

Après homogénéisation, l'émulsion est laissée à refroidir jusqu'à température ambiante, puis neutralisée, par exemple par ajout d'hydroxyde de sodium, afin d'obtenir un pH de 7-7,5.

On peut alors stériliser l'émulsion, par exemple par traitement thermique à 120-130°C pendant 8-30 minutes, puis la conditionner, de manière aseptique, dans des boîtes, des briques ou des flacons.

On obtient ainsi une émulsion huile-dans-eau, présentant un taux de lipides d'au moins 10%, qui peut conserver sa stabilité pendant une longue période, et qui comprend au moins 0,4% de composé émulsifiant comprenant PC et PE, en un rapport PC/PE compris entre 2 et 10.

Cette émulsion peut être utilisée dans le domaine de l'alimentation parentérale, telle quelle ou après dilution.

Ainsi, dans une forme particulière d'application, on peut préparer, à partir de cette émulsion, une composition nutritive comprenant 1 partie de ladite émulsion et 0-15 parties d'eau pouvant contenir une source d'acides aminés.

On peut ajouter ladite source d'acides aminés, de préférence en solution aqueuse, à l'émulsion préparée, avant ou après homogénéisation et/ou stérilisation.

On peut également ajouter ladite source d'acides aminés à la phase aqueuse, avant préparation de l'émulsion.

La source d'acides aminés peut être constituée, par exemple, d'acides aminés et/ou de peptides.

On a constaté que, grâce à la présente invention, on pouvait ajouter jusqu'à 6% d'une source d'acides aminés à une émulsion à 10 ou 20% de lipides, sans entraîner de déstabilisation de la composition après stérilisation.

On obtient alors, après stérilisation, une composition nutritive stable, comprenant des acides aminés, susceptible d'être utilisée dans le domaine de l'alimentation parentérale.

La présente invention est illustrée plus en détails dans les exemples ci-après, dans lesquels les mesures sont effectuées de la manière suivante:

1) Mesure d'osmolalité

On entend par osmolalité, le nombre de moles osmotiquement actives par kilogramme de produit pris comme référence, dans le cas présent par kilogramme d'eau (unité Osm/kg d'eau).

On la mesure à l'aide d'un osmomètre Roebling, par mesure de l'abaissement cryoscopique de la solution, en prenant l'eau pure comme référence.

2) Mesure de la teneur en matière grasse

On détermine la teneur en matière grasse de la composition par mesure de la quantité de lipides, effectuée de la manière suivante:
- on prélève une quantité précise, comprise entre 10 et 20 g, de liquide à analyser,
- on dilue ce liquide avec 20 ml d'une solution aqueuse de NaCl à 10%,
- on ajuste à pH 3 par ajout d'une solution aqueuse de HCl 0,1 N,
- on extrait quatre fois les lipides avec 50 ml d'une solution contenant 3 parties de n-hexane et 2 parties d'isopropanol,
- on lave la phase organique obtenue avec 30 ml de NaCl 10%, et la phase aqueuse obtenue avec 30 ml de n-hexane,
- les phases organiques sont réunies et séchées sur du sulfate de sodium anhydre, puis filtrées.

Les solvants organiques sont alors évaporés sous pression réduite et l'on obtient par pesée la quantité de lipides. La teneur T en matière grasse, exprimée en %, est obtenue en faisant le rapport entre cette quantité de lipides pesée et la quantité de liquide initialement prélevée.

3) Détermination de l'indice de stabilité

Afin de caractériser quantitativement la stabilité physique des compositions nutritives préparées, on détermine l'indice de stabilité (IS), de la façon suivante:
- on prélève un premier échantillon de la composition préparée et l'on en détermine la teneur en matière grasse T1,
- on prélève un second échantillon que l'on soumet à une centrifugation, à 25 °C, à 1000 tours/min pendant 15 minutes; il se produit généralement une séparation en deux phases plus ou moins distinctes;
- on détermine la teneur en matière grasse T 2 de la partie inférieure de l'échantillon centrifugé et,
- on obtient l'indice de stabilité IS (en%) en faisant le rapport

$$\frac{T\ 2\ x\ 100}{T\ 1}$$

4) Préparation des composés PC/PE

Certains des composés émulsifiants employés dans les exemples suivants ont été préparés de la manière suivante :
- on pèse une quantité Q1 de lécithine présentant un rapport PC/PE de 4/1, que l'on dissout dans 100 ml d'un mélange de dichlorométhane-méthanol 2:1,
- on ajoute une quantité Q2 de lécithine présentant un rapport PC/PE de 10/8 (ou une quantité Q3 de PE pure) et l'on mélange bien,
- on évapore à sec le solvant,
- on ajoute 100 ml d'eau, on redisperse sous agitation puis on lyophilise le produit obtenu.
Quantités à ajouter pour les différents mélanges PC/PE:

| PC/PE | 2,5/1 | 6/1 | 8/1 |
|---|---|---|---|
| Q1 | 1g | 2g | 1g |
| Q2 | | 1,832g | 3,223g |
| Q3 | 0,125g | | |

4

Exemple 1

On prépare une phase aqueuse en mélangeant, à 45°C et sous agitation, 14,6 ml d'eau, 0,4 ml de glycérol et de la lécithine d'oeuf, présentant un rapport PC/PE de 4,1/1, en une quantité telle que définie dans le tableau suivant.

On ajoute lentement à cette phase aqueuse, 3,8 g d'une huile de soja, tout en maintenant une agitation constante et la température à environ 45°C.

On obtient ainsi une émulsion à 20% de lipides, qui est homogénéisée après cinq passages dans un appareil adéquat, de type "Microfluidizer", sous une pression de 1100 bar et entre 20°C et 50°C, de manière à obtenir une taille moyenne des gouttelettes de phase lipidique entre 250 et 950 nm.

On ajuste le pH de l'émulsion ainsi préparée à 7,4, à l'aide d'hydroxyde de sodium 0,5 N, puis l'on dispose cette émulsion dans des flacons de 100 ml qui sont stérilisés à 126°C pendant 9 minutes.

On détermine l'osmolalité et le pH de l'émulsion avant et après stérilisation. On effectue également une observation visuelle de la stabilité de l'émulsion.

On obtient les résultats suivants :

|        | A1   | A2   | A3   | A4   | A5   | A6   |
|--------|------|------|------|------|------|------|
| %      | 1,28 | 1,05 | 0,83 | 0,62 | 0,41 | 0,21 |
| osmo 1 | 342  | 346  | 351  | 327  | 353  | 327  |
| pH 1   | 3,87 | 3,99 | 4,17 | 4,67 | 5,06 | 5,82 |
| osmo 2 | 343  | 349  | 353  | 325  | 353  | 328  |
| pH 2   | 4,76 | 5,08 | 5,08 | 5,30 | 5,29 | 5,23 |
| T0     | +    | +    | +    | +    | +    | 0    |
| T1     | +    | +    | +    | +    | +    | -    |
| T2     | +    | +    | +    | +    | 1    | -    |

% : pourcentage de lécithine dans l'émulsion osmo 1 : osmolalité avant stérilisation (en mOsm/kg) pH 1 : pH avant stérilisation osmo 2 : osmolalité après stérilisation (en mOsm/kg) pH 2 : pH après stérilisation T0 : observation juste après préparation de l'émulsion T1 : observation après 1 mois de stockage à température ambiante.

T2 : observation après 2 mois de stockage à température ambiante.

 + : émulsion stable 0 : légère trace de séparation, émulsion de nouveau homogène après agitation 1 : début de séparation, de floculation et/ou de sédimentation

- : séparation nette, émulsion cassée de manière irréversible

On remarque donc que l'ajout d'une quantité de 0,21% de lécithine présentant un rapport PC/PE de 4,1/1 n'est pas suffisante pour permettre l'obtention d'une émulsion qui reste stable après un ou deux mois de stockage.

L'ajout d'une quantité de 0,4% d'une lécithine présentant un rapport PC/PE de 4,1/1 est suffisant pour maintenir l'émulsion stable pendant au moins deux mois.

On prépare ensuite une composition nutritive.

On prépare tout d'abord une solution aqueuse contenant des acides aminés, dont la L-leucine, la glycine, la L-alanine et la L-proline, en une quantité totale de 6,0 g dans 75 ml d'eau.

On ajoute 81 g de cette solution aqueuse à 19 g de l'émulsion préparée ci-dessus.

On stérilise la composition nutritive obtenue, à 126°C pendant 1 minute.

On détermine l'osmolalité et le pH de ladite composition nutritive avant et après stérilisation. On effectue également une observation visuelle de la stabilité de la composition.

On obtient les résultats suivants :

| | B1 | B2 | B3 | B4 | B5 | B6 |
|---|---|---|---|---|---|---|
| % | 0,24 | 0,20 | 0,16 | 0,12 | 0,08 | 0,04 |
| osmo 1 | 753 | 784 | 611 | 752 | 755 | 746 |
| pH 1 | 8,01 | 8,09 | 8,08 | 8,16 | 8,25 | 8,35 |
| osmo 2 | 756 | 789 | 614 | 752 | 758 | 744 |
| pH 2 | 8,07 | 8,09 | 7,99 | 8,02 | 8,21 | 8,33 |
| T0 | + | + | + | + | + | 0 |
| T1 | 0 | 0 | 0 | 1 | 1 | - |
| T2 | 0 | 0 | 0 | 1 | - | - |

% : pourcentage de lécithine dans la composition nutritive finale osmo 1 : osmolalité avant stérilisation (en mOsm/kg) osmo 2 : osmolalité après stérilisation (en mOsm/kg) pH 1/pH 2 : pH avant/après stérilisation T0 : observation juste après préparation de la composition T1/T2 : observation après 1 et 2 mois de stockage à température ambiante.

+ : composition nutritive stable 0 : légère trace de séparation, composition de nouveau homogène après agitation 1 : début de séparation, de floculation et/ou de sédimentation

- : séparation nette, composition cassée de manière irréversible

On remarque donc que l'ajout d'une quantité de 0,04% de lécithine présentant un rapport PC/PE de 4,1/1 n'est pas suffisant pour permettre l'obtention d'une composition nutritive stable.

L'ajout d'une quantité de 0,08% de lécithine présentant un rapport PC/PE de 4,1/1 est suffisant pour permettre l'obtention d'une composition nutritive à 20% de lipides et contenant des acides aminés, stable, mais pas pour la maintenir stable pendant plusieurs mois de stockage.

Exemple 2

On prépare une phase aqueuse en mélangeant, à 45°C et sous agitation, 14,6 ml d'eau, 0,4 ml de glycérol et de la lécithine d'oeuf, présentant un rapport PC/PE de 4,8/1, en une quantité telle que définie dans le tableau suivant.

On ajoute lentement à cette phase aqueuse, 3,8 g d'une huile de soja, tout en maintenant une agitation constante et la température à environ 45°C.

On obtient ainsi une émulsion à 20% de lipides, qui est homogénéisée de la même manière que précédemment de manière à obtenir une taille moyenne des gouttelettes de phase lipidique entre 250 et 850 nm.

On ajuste le pH de l'émulsion ainsi préparée à 7,4, à l'aide d'hydroxyde de sodium 0,5 N, puis l'on dispose cette émulsion dans des flacons de 100 ml qui sont stérilisés à 126°C pendant 9 minutes.

On détermine l'osmolalité et le pH de l'émulsion avant et après stérilisation. On effectue également une observation visuelle de la stabilité de l'émulsion.

On obtient les résultats suivants :

|  | C1 | C2 | C3 | C4 | C5 | C6 |
|---|---|---|---|---|---|---|
| % | 1,28 | 1,05 | 0,83 | 0,62 | 0,41 | 0,21 |
| osmo 1 | 333 | 321 | 331 | 341 | 340 | 376 |
| pH 1 | 4,26 | 4,58 | 4,59 | 5,18 | 5,05 | 6,20 |
| osmo 2 | 331 | 325 | 333 | 342 | 339 | 377 |
| pH 2 | 5,33 | 5,68 | 6,02 | 5,41 | 5,52 | 5,30 |
| T0 | + | + | + | + | + | 0 |
| T1 | + | + | + | + | + | - |
| T2 | + | + | + | 1 | - | - |

% : pourcentage de lécithine dans l'émulsion osmo 1 : osmolalité avant stérilisation (en mOsm/kg) osmo 2 : osmolalité après stérilisation (en mOsm/kg) pH 1/pH 2 : pH avant/après stérilisation T0 : observation juste après préparation de l'émulsion T1/T2 : observation après 1 et 2 mois de stockage à température ambiante.

+ : émulsion stable 0 : légère trace de séparation, émulsion de nouveau homogène après agitation 1 : début de séparation, de floculation et/ou de sédimentation

- : séparation nette, émulsion cassée de manière irréversible

On remarque que l'ajout d'une quantité de 0,21% de lécithine présentant un rapport PC/PE de 4,8/1 n'est pas suffisant pour permettre l'obtention d'une émulsion qui reste stable après un ou deux mois de stockage.

L'ajout d'une quantité de 0,4% de lécithine présentant un rapport PC/PE de 4,8/1 est suffisant pour maintenir l'émulsion stable pendant au moins un mois.

On prépare ensuite une composition nutritive.

On prépare tout d'abord une solution aqueuse contenant des acides aminés, dont la L-leucine, la glycine, la L-alanine et la L-proline, en une quantité totale de 6,0 g dans 75 ml d'eau.

On ajoute 81 g de cette solution aqueuse à 19 g de l'émulsion préparée ci-dessus.

On stérilise la composition nutritive obtenue, à 126°C pendant une minute.

On détermine l'osmolalité et le pH de ladite composition nutritive avant et après stérilisation. On effectue également une observation visuelle de la stabilité de la composition.

On obtient les résultats suivants :

|  | D1 | D2 | D3 | D4 | D5 | D6 |
|---|---|---|---|---|---|---|
| % | 0,24 | 0,20 | 0,16 | 0,12 | 0,08 | 0,04 |
| osmo 1 | 797 | 780 | 627 | 747 | 746 | 761 |
| pH 1 | 8,06 | 8,08 | 8,05 | 8,22 | 8,30 | 8,29 |
| osmo 2 | 751 | 779 | 625 | 750 | 758 | 758 |
| pH 2 | 8,01 | 8,07 | 8,00 | 8,31 | 8,30 | 8,26 |
| T0 | + | + | + | + | + | 0 |
| T1 | 0 | 0 | 0 | 1 | - | - |
| T2 | 0 | 1 | 1 | 1 | - | - |

% : pourcentage de lécithine dans la composition nutritive finale osmo 1 : osmolalité avant stérilisation (en mOsm/kg) osmo 2 : osmolalité après stérilisation (en mOsm/kg) pH 1/pH 2 : pH avant/après stérilisation T0 : observation juste après préparation de la composition nutritive T1/T2 : observation après 1 et 2 mois de stockage à température ambiante

+ : composition nutritive stable 0 : légère trace de séparation, composition de nouveau homogène après agitation 1 : début de séparation, de floculation et/ou de sédimentation

- : séparation nette, composition cassée de manière irréversible

L'ajout d'une quantité de 0,04% de lécithine présentant un rapport PC/PE de 4,8/1 n'est pas suffisant pour permettre l'obtention d'une composition nutritive stable.

L'ajout d'une quantité de 0,12% de lécithine présentant un rapport PC/PE de 4,8/1 est suffisant pour permettre l'obtention d'une composition nutritive à 20% de lipides et contenant des acides aminés, stable.

Exemple 3

On prépare une phase aqueuse en mélangeant, à 45°C et sous agitation, 14,6 ml d'eau, 0,4 ml de glycérol et un composé émulsifiant présentant un rapport PC/PE aux concentrations définies dans le tableau suivant.

On ajoute lentement à cette phase aqueuse, 3,8 g d'une huile de soja, tout en maintenant une agitation constante et la température à environ 45°C.

On obtient ainsi une émulsion à 20% de lipides, qui est homogénéisée de la même manière que précédemment.

On ajuste le pH de l'émulsion ainsi préparée à 7,4, à l'aide d'hydroxyde de sodium 0,5 N.

On prépare ensuite une composition nutritive.

On prépare tout d'abord une solution aqueuse contenant des acides aminés, dont la L-leucine, la glycine, la L-alanine et la L-proline, en une quantité totale de 6,0 g dans 75 ml d'eau.

On ajoute 81 g de cette solution aqueuse à 19 g de l'émulsion préparée ci-dessus.

On stérilise la composition nutritive obtenue, à 126°C pendant une minute.

On détermine l'osmolalité et le pH de ladite composition nutritive avant et après stérilisation.

On effectue également une observation visuelle de la stabilité de la composition et l'on détermine l'indice de stabilité.

On obtient les résultats suivants :

Observations

| %    | PC/PE  | osmo 1 | osmo 2 | pH 1 | pH 2 | T0 | T1 | T2 | IS |
|------|--------|--------|--------|------|------|----|----|----|----|
| 0,16 | 2,5/1  | 709    | 711    | 7,92 | 7,88 | +  | 1  |    | 87 |
| 0,04 | 4,1/1  | 746    | 745    | 8,35 | 8,33 | 0  | –  | –  |    |
| 0,08 | "      | 755    | 758    | 8,25 | 8,21 | +  | 1  | –  | 72 |
| 0,12 | "      | 752    | 751    | 8,16 | 8,02 | +  | 1  | 1  | 92 |
| 0,16 | "      | 611    | 614    | 8,08 | 7,99 | +  | 0  | 0  | 95 |
| 0,20 | "      | 784    | 789    | 8,09 | 8,09 | +  | 0  | 0  | 96 |
| 0,24 | "      | 754    | 757    | 8,01 | 8,07 | +  | 0  | 0  |    |
| 0,04 | 4,8/1  | 761    | 758    | 8,29 | 8,26 | 0  | –  | –  |    |
| 0,08 | "      | 746    | 749    | 8,30 | 8,30 | +  | –  | –  |    |
| 0,12 | "      | 747    | 750    | 8,22 | 8,31 | +  | 1  | 1  | 89 |
| 0,16 | "      | 627    | 625    | 8,05 | 8,00 | +  | 0  | 1  | 90 |
| 0,20 | "      | 780    | 779    | 8,03 | 8,07 | +  | 0  | 1  | 93 |
| 0,24 | "      | 797    | 751    | 8,06 | 8,01 | +  | 0  | 0  |    |
| 0,16 | 6,0/1  | 709    | 709    | 8,02 | 7,82 | +  | 1  |    | 87 |
| 0,16 | 8,0/1  | 697    | 698    | 7,91 | 7,80 | +  | 1  |    | 87 |
| 0,04 | 10,8/1 | 705    | 701    | 8,00 | 7,89 | 1  | –  |    |    |
| 0,08 | "      | 694    | 697    | 8,06 | 8,03 | 0  | –  |    |    |
| 0,12 | "      | 702    | 699    | 8,03 | 7,99 | 0  | –  |    | 34 |
| 0,16 | "      | 679    | 680    | 8,08 | 8,03 | +  | –  |    | 34 |
| 0,20 | "      | 684    | 676    | 7,80 | 7,92 | +  | –  |    | 24 |
| 0,24 | "      | 737    | 736    | 8,18 | 7,92 | +  | –  |    |    |

% : pourcentage de lécithine dans la composition nutritive
     finale

PC/PE : rapport PC/PE du composé émulsifiant

osmo 1 : osmolalité avant stérilisation (en mOsm/kg) osmo 2 : osmolalité après stérilisation (en mOsm/kg) pH 1/pH 2 : pH avant/après stérilisation T0 : observation juste après préparation de la composition
nutritive finale

T1/T2 : observation après 1 et 2 mois de stockage à température ambiante.


+ : composition nutritive stable 0 : légère trace de séparation, composition de nouveau
homogène après agitation 1 : début de séparation, de floculation et/ou de
sédimentation

- : séparation nette, composition cassée de manière
irréversible

IS : indice de stabilité


On remarque donc qu'une quantité de 0,08% de composé émulsifiant est suffisante pour maintenir stable une composition nutritive à 20% de lipides et présentant un taux de matière sèche d'environ 10%, si ledit composé émulsifiant présente un rapport PC/PE compris entre 2 et 10.

Exemple 4

On prépare une phase aqueuse en mélangeant, à 45°C et sous agitation, 14,6 ml d'eau, 0,4 ml de glycérol et un composé émulsifiant présentant un rapport PC/PE aux concentrations définies dans le tableau suivant.
On ajoute lentement à cette phase aqueuse, 3,8 g d'une huile de soja, tout en maintenant une agitation constante et la température à environ 45°C.
On obtient ainsi une émulsion à 20% de lipides, qui est homogénéisée de la même manière que précédemment.
On ajuste le pH de l'émulsion ainsi préparée à 7,4, à l'aide d'hydroxyde de sodium 0,5 N.
On prépare ensuite une composition nutritive.
On prépare tout d'abord une solution aqueuse contenant des acides aminés, dont la L-leucine, la glycine, la L-alanine et la L-proline, en une quantité totale de 6,0 g dans 75 ml d'eau.
On ajoute 81 g de cette solution aqueuse à 19 g de l'émulsion préparée ci-dessus.
On stérilise la composition nutritive obtenue, à 126°C pendant une minute.
On détermine l'indice de stabilité pour les différentes compositions nutritives, en fonction de la durée de stockage.
On obtient les résultats suivants :

| % | PC/PE | Indice de Stabilité IS (%) | | | |
|---|---|---|---|---|---|
| | | T0 | T1 | T2 | T3 |
| 0,08 | 4,1 | 76 | 73 | 77 | 76 |
| 0,12 | 4,8 | 94 | 87 | 86 | 84 |
| 0,20 | 4,8 | 85 | 85 | 85 | 83 |
| 0,20 | 10,8 | 55 | 54 | 54 | 56 |
| % : pourcentage de composé émulsifiant dans la composition nutritive finale | | | | | |

On constate donc que ces compositions nutritives présentent un indice de stabilité relativement constant et élevé, ce qui signifie qu'elles restent stables pendant au moins trois mois.

Exemple 5

On prépare une phase aqueuse en mélangeant, à 45°C et sous agitation, 14,6 ml d'eau, 0,4 ml de glycérol et un composé émulsifiant présentant un rapport PC/PE tel que défini dans le tableau suivant, à une concentration de 0,16% dans la composition finale (0,83% dans l'émulsion).

On ajoute lentement à cette phase aqueuse, 3,8 g d'une huile de soja, tout en maintenant une agitation constante et la température à environ 45°C.

On obtient ainsi une émulsion à 20% de lipides, qui est homogénéisée de la même manière que précédemment.

On ajuste le pH de l'émulsion ainsi préparée à 7,4, à l'aide d'hydroxyde de sodium 0,5 N.

On prépare ensuite une composition nutritive.

On prépare tout d'abord une solution aqueuse contenant des acides aminés, dont la L-leucine, la glycine, la L-alanine et la L-proline, en une quantité totale de 6,0 g dans 75 ml d'eau.

On ajoute 81 g de cette solution aqueuse à 19 g de l'émulsion préparée ci-dessus.

On stérilise la composition obtenue, à 126°C pendant une minute.

On détermine l'indice de stabilité pour les différentes compositions nutritives obtenues.

On obtient les résultats suivants :

| PC/PE | Lipides totaux % | IS % |
|---|---|---|
| 0,5 | 4,2 | 25 |
| 2,5 | 4,5 | 87 |
| 4,1 | 4,3 | 95 |
| 4,8 | 4,2 | 90 |
| 6,0 | 3,9 | 87 |
| 8,0 | 3,9 | 87 |
| 10,8 | 4,1 | 34 |

L'indice de stabilité IS est déterminé après 15 minutes de centrifugation à 2000 tours par minute.

L'indice de stabilité est relativement élevé, sauf pour les compositions contenant un composé émulsifiant présentant un rapport PC/PE qui n'est pas compris entre 2 et 10.

**Revendications**

1. Composition se présentant sous forme d'une émulsion huile-dans eau, comprenant, en poids, au moins 10% de lipides et au moins 0,4% d'un composé émulsifiant comprenant de la phosphatidyle choline (PC) et de la phosphatidyle éthanolamine (PE), en un rapport PC/PE compris entre 2 et 10.

2. Composition nutritive comprenant 1 partie en poids de la composition selon la revendication 1 et 0-15 parties d'eau pouvant contenir une source d'acides aminés.

3. Procédé de préparation d'une composition se présentant sous forme d'une émulsion huile-dans-eau, dans lequel on prépare une phase aqueuse et une phase lipidique, on ajoute à l'une et/ou l'autre des phases au moins 0,4% en poids d'un composé émulsifiant comprenant de la phosphatidyle choline (PC) et de la phosphatidyle éthanolamine (PE), en un rapport PC/PE compris entre 2 et 10, on mélange les deux phases résultantes de manière à obtenir une émulsion comprenant au moins 10% en poids de lipides, et l'on stérilise l'émulsion.

4. Procédé de préparation selon la revendication 3, dans lequel on ajoute à 1 partie en poids de l'émulsion avant ou après sa stérilisation, 0-15 parties d'eau pouvant contenir une source d'acides aminés, puis l'on stérilise la composition nutritive ainsi obtenue.

5. Utilisation des compositions selon les revendications 1 et 2 dans le domaine de l'alimentation parentérale.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X <br> Y | US-A-4 563 354 (S.S CHANG) <br><br> * revendications 6,15,18-20 * <br> * colonne 6, ligne 20 - ligne 55 * <br> * tableaux 6,7 * <br> --- | 1,2,5 <br> 3,4 | A23L1/30 <br> A23J7/00 <br> A61K9/00 <br> A23L1/035 |
| X | EP-A-0 115 981 (SYNTHELABO) <br> * page 2, ligne 22 - ligne 26 * <br> * page 3, ligne 11 - page 4, ligne 21 * <br> --- | 1,5 | |
| Y,D | REVUE FRANCAISE DES CORPS GRAS <br> vol. 31, no. 10 , 1984 , PARIS FR <br> pages 391 - 394 <br> G. MASSON <br> * page 391, colonne de droite, alinéa 2 - page 392, colonne de gauche, alinéa 13 * <br> * page 391, colonne de droite * <br> --- | 3 | |
| Y | EP-A-0 312 612 (OTSUKA PHARMACEUTICAL CO.) <br> * page 14, alinéa 3 - page 16, alinéa 1 * <br> * exemple 1 * <br> --- | 4 | |
| A | JOURNAL OF PHARMACEUTICAL SCIENCES <br> vol. 73, no. 1 , Janvier 1984 , <br> WASHINGTON, USA <br> pages 91 - 94 <br> A. TAKAMURA <br> * page 91, colonne de droite, alinéa 2 -alinéa 3 * <br> --- | 4 | **DOMAINES TECHNIQUES RECHERCHES (Int.Cl.5)** <br><br> A23L <br> A61K |
| A | DE-A-29 48 607 (CHEMISCHE FABRIK DR. MEYER & CO.) <br> * revendications 1,2,12,13 * <br> * page 8, ligne 8 - ligne 15 * <br> * page 14, ligne 10 - ligne 15 * <br> * page 15, ligne 1 - ligne 15 * <br> --- <br><br> -/-- | 1 | |

Le présent rapport a été établi pour toutes les revendications

| Lieu de la recherche | Date d'achèvement de la recherche | Examinateur |
|---|---|---|
| LA HAYE | 24 Janvier 1994 | Vuillamy, V |

Office européen
des brevets

**RAPPORT DE RECHERCHE EUROPEENNE**

Numero de la demande

EP 93 11 2386

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | WO-A-88 08301 (BAXTER TRAVENOL LABORATORIES)<br>* revendications 1-10 *<br>* page 1, ligne 1 - page 5, ligne 29 *<br>----- | 1,2 | |
| | | | **DOMAINES TECHNIQUES RECHERCHES (Int.Cl.5)** |

Le présent rapport a été établi pour toutes les revendications

| Lieu de la recherche | Date d'achèvement de la recherche | Examinateur |
|---|---|---|
| LA HAYE | 24 Janvier 1994 | Vuillamy, V |